(19) Europäisches Patentamt
European Patent Office
Office européen des brevets

(11) Veröffentlichungsnummer: **0 279 325 B1**

(12) # EUROPÄISCHE PATENTSCHRIFT

(45) Veröffentlichungstag der Patentschrift: **16.05.90**

(51) Int. Cl.[5]: **C07C 69/743**, A01N 53/00

(21) Anmeldenummer: **88101835.2**

(22) Anmeldetag: **09.02.88**

(54) **(+)1 R-trans-2,2-Dimethyl-3-(2,2-dichlorvinyl)-cyclopropancarbonsäure-2,3,5,6-tetrafluorbenzylester.**

(30) Priorität: **19.02.87 DE 3705224**

(43) Veröffentlichungstag der Anmeldung:
**24.08.88 Patentblatt 88/34**

(45) Bekanntmachung des Hinweises auf die Patenterteilung:
**16.05.90 Patentblatt 90/20**

(84) Benannte Vertragsstaaten:
**AT BE CH DE ES FR GB GR IT LI NL SE**

(56) Entgegenhaltungen:
**EP-A- 0 017 810**
**EP-A- 0 200 943**
**FR-A- 2 379 506**

(73) Patentinhaber: **BAYER AG,**
**D-5090 Leverkusen 1 Bayerwerk(DE)**

(72) Erfinder: **Naumann, Klaus, Dr.,**
**Richard-Wagner-Strasse 9, D-5090 Leverkusen(DE)**
Erfinder: **Behrenz, Wolfgang, Dr., Untergruendemich 14,**
**D-5063 Overath(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 279 325 B1

**Beschreibung**

Die vorliegende Erfindung betrifft den (+)1R-trans-2,2-Dimethyl-3-(2,2-dichlorvinyl)-cyclopropancarbonsäure-2,3,5,6-tetrafluorbenzylester, Verfahren zu seiner Herstellung sowie seine Verwendung als Insektizid.

Es ist bereits bekannt, daß Ester der 2,2-Dimethyl-3-(2,2-dichlorvinylcyclopropancarbonsäure) mit polyfluorierten Benzylalkoholen insektizide Eigenschaften aufweisen (siehe dazu DE-PS 2 658 074 bzw. GB-PS 1 567 820). Der Pentafluorbenzylester zeigt dabei eine überragende Wirkung, da bereits ein Fünfzehntel von ihm Fliegen in der gleichen Zeit abtötete wie ein Gemisch aus gleichen Teilen des 2,3,5,6-Tetrafluorbenzyl- und des 3,5,6-Trifluorbenzylesters. De Tetrafluorbenzylester allein zeigt ebenfalls eine gute insektizide Wirkung.

Es ist weiter bekannt, daß der (-)1R-trans-2,2-Dimethyl-3-(2,2-dichlorvinyl)-cyclopropancarbonsäurepentafluorbenzylester zur Bekämpfung von Haushalt-, Hygiene- und Vorratsschädlingen hervorragend geeignet ist. (Behrenz, Naumann 1982). Aus der gleichen Veröffentlichung ist aber auch bekannt, daß dieser Wirkstoff eine relativ hohe Säugetiertoxizität besitzt, ($LD_{50}$ oral in mg/kg Ratte: 90-105). Eine noch höhere Toxizität besitzt auch das cis/transGemisch des entspr. 2,3,5,6-Tetrafluor benzylesters ($LD_{50}$ oral in mg/kg männliche Ratte: 10-25). Die Verwendung solch toxischer wirkstoffe in Haushalts-, Hygiene und Vorratsschutzmitteln braucht dennoch nicht prohibitiz zu sein, wenn sie in entsprechend geringen Dosierungen eingesetzt werden können. Es ist jedoch Aufgabe der Forschung, nach immer ungiftigeren Substanzen zu suchen, die eine immer größere Spanne zwischen wirksamer Dosis auf den Schädling einerseits und toxischer Wirkung für Mensch und Tier andererseits besitzen, d.h. die einen sehr günstigen therapeutischen Index besitzen, weil sich hierdurch die Sicherheit in der Anwendung solcher Verbindungen erhöht.

Es wurde nun der neue (+)1R-trans-2,2-Dimethyl-3-(2,2-dichlorvinyl)-cyclopropancarbonsäure-2,3,5,6-tetrafluorbenzylester der Formel (I)

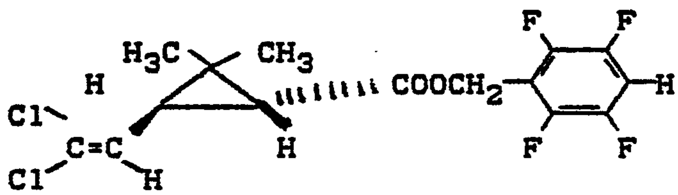
(I)

gefunden.

Man erhält den (+)1R-trans-2,2-Dimethyl-3-(2,2-dichlorvinyl)-cyclopropancarbonsäure-2,3,5,6-tetrafluorbenzylester der Formel (I)

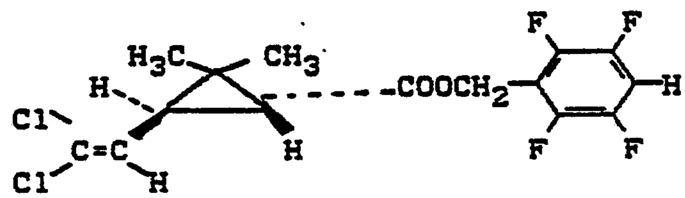
(I)

wenn man

a) entweder

(+)1R-trans-Permethrinsäurechlorid der Formel

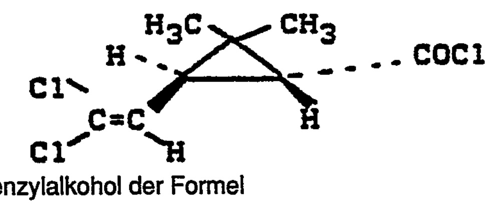
(II)

mit 2,3,5,6-Tetrafluorbenzylalkohol der Formel

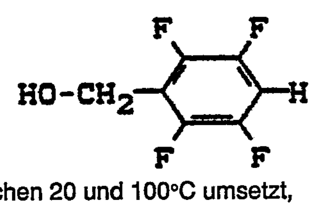
(III)

bei Temperaturen zwischen 20 und 100°C umsetzt,

oder

b) das Salz der (+)1R-trans-Permethrinsäure der Formel

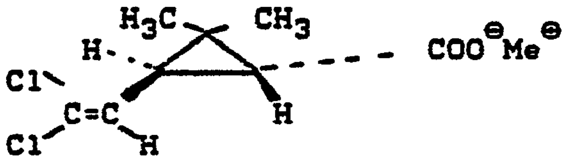 (IV)

worin Me⊕ für ein einwertiges Kation steht, mit einer Verbindung der Formel

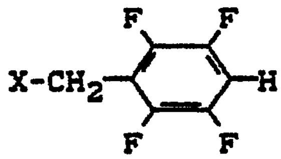 (V)

worin

X für einen anionisch abspaltbaren Rest steht,

umsetzt.

Der neue (+)1R-trans-2,2-Dimethyl-3-(2,2-dichlorvinyl)-cyclopropancarbonsäure-2,3,5,6-tetrafluorbenzylester zeigt einen im überraschenden Maße günstigen therapeutischen Index, da er bei hoher Wirksamkeit eine extrem niedrige Säugetiertoxizität besitzt ($LD_{50}$ oral in mg/kg männliche Ratte: größer als 5000!).

Seine Säugetiertoxizität ist also mehr als 250 mal niedriger als die des cis/trans-2,2-Dimethyl-3-(2,2-dichlorvinyl)-cyclopropancarbonsäure-tetrafluorbenzyl- und 50 mal niedriger als die des (-)1R-trans-2,2-Dimethyl-3-(2,2-dichlorvinyl)-cyclopropancarbonsäurepentafluorbenzylesters. Dies wäre allein noch nicht so überraschend, wenn die beanspruchte neue Verbindung in gleichem Maße auch an Wirksamkeit gegen Schadorganismen verlieren würden. Dies ist jedoch nicht der Fall. Im Gegenteil besitzt der toxischere cis/trans-2,2-Dimethyl-3-(2,2-dichlorvinyl)-cyclopropancarbonsäure-tetrafluorbenzylester eine geringere biologische Wirkung als der weniger toxische, erfindungsgemäße (+)1R-trans-2,2-Dimethyl-3-(2,2-dichlorvinyl)-cyclopropancarbonsäuretetrafluorbenzylester. Im Vergleich zu dem (-)1R-trans-3 (2,2-dichlorvinyl)-cyclopropancarbonsäure-pentafluorbenzylester besitzt der (+)1R-trans-2,2-Dimethyl-3-(2,2-dichlorvinyl)-cyclopropancarbonsäuretetrafluorbenzylester der Formel (I) in gleicher oder nur geringfügig höherer Dosierung eine vergleichbare insektizide Wirksamkeit.

Die Bereitstellung des neuen (+)1R-trans-2,2-Dimethyl-3-(2,2-dichorvinyl)-cyclopropancarbonsäure-2,3,5,6-tetrafluorbenzylesters stellt somit eine große Bereicherung des Standes der Technik dar.

Die Herstellung des (+)1R-trans-2,2-Dimethyl-3-(2,2-dichlorvinyl)-cyclopropancarbonsäure-2,3,5,6-tetrafluorbenzylesters kann durch die folgenden Formelschemata wiedergegeben werden:

a)

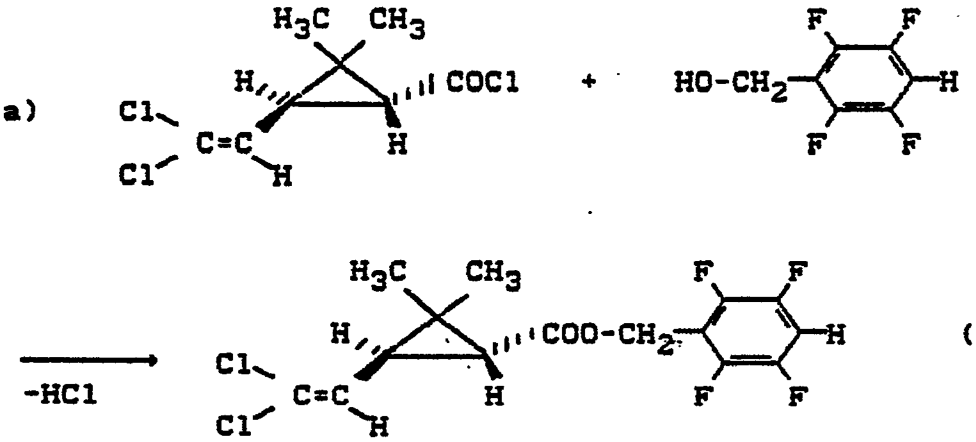


(I)

b)

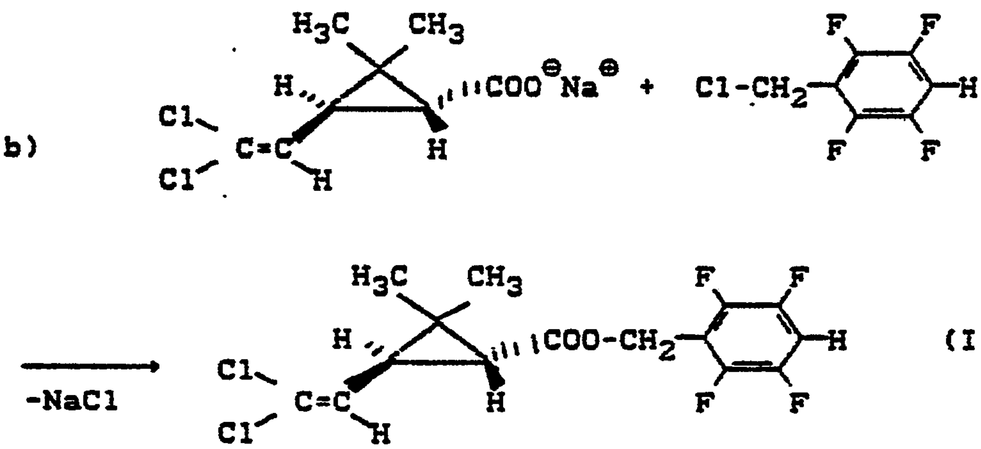


(I)

Bei der Verfahrensvariante a) setzt man das aus Pesticide Science 1974, 796 bekannte (+)1R-trans-Permethinsäurechlorid in Gegenwart oder in Abwesenheit von Lösungsmitteln und gegebenenfalls in Gegenwart von Säurebindungsmitteln mit dem aus J. Chem. Soc. C. 1968, 1575 bekannten 2,3,5,5-Tetrafluorbenzylalkohol bei Temperaturen zwischen 20 und 100°C um.

Hierbei werden die Komponenten vorzugsweise in Abwesenheit von Lösungsmitteln und von Säurebindungsmitteln bei Temperaturen zwischen 30 und 80°C umgesetzt, wobei man die entstehende leichtflüchtige Komponente (vorzugsweise Chlorwasserstoffgas) entweichen läßt und anschließend das Reaktionsprodukt vorzugsweise destillativ aufarbeitet. Die Ausgangskomponenten der Verfahrensvariante a) werden vorzugsweise in äquimolaren Mengen eingesetzt.

Bei der Verfahrensvariante b) setzt man die Salze (insbesondere die Alkalisalze) der (+)1R-trans-Permethinsäure ebenfalls vorzugweise in äquimolaren Mengen mit vorzugsweise 2,3,5,6-Tetrafluorbenzylchlorid, -bromid oder -tosylat im Sinne einer Veresterungsreaktion um, analog der Verfahrensweise aus Synthesis 1985, 805, in Gegenwart oder in Abwesenheit eines Alkylierungskatalysators.

Bei den Ausgangsverbindungen der Formel (IV) steht $Me^{\oplus}$ vorzugsweise für ein einwertiges Metallkation und insbesondere für ein Alkalimetallkation, beispielsweise sei $Na^{\oplus}$ genannt.

Bei den Ausgangsverbindungen der Formel (V) steht X, vorzugsweise für Halogen, insbesondere für Chlor oder Brom oder für den Tosylatrest (=Rest der p-Toluolsulfonsäure).

Die Umsetzung gemäß Verfahrensvariante b) wird vorzugsweise in einem Lösungsmittel, insbesondere einem für die Umsetzung inerten, polaren organischen Lösungsmittel durchgeführt. Beispielhaft seien genannt: Acetonitril, Aceton, Dimethylformamid.

Die Aufarbeitung der entstehenden Verbindung der Formel (I) geschieht auch hier vorzugsweise destillativ.

Beide Varianten a) und b) werden vorzugsweise bei Normaldruck durchgeführt.

Der erfindungsgemäße Wirkstoff eignet sich zur Bekämpfung von tierischen Schädlingen, insbesondere von Insekten, die im Haushalt oder als Hygiene-, Vorrats-schädlinge vorkommen. Er ist gegen normalsensible und/oder resistente Arten sowie gegen alle oder einzelne Entwicklungsstadien wirksam. Zu den oben erwähnten Schädlingen gehören:

Aus der Ordnung der Thysanura z.B. Lepisma saccharina.
Aus der Ordnung der Orthoptera z.B. Blatta orientalis, Periplaneta americana, Leucophaea maderae, Blattella germanica, Acheta domesticus.
Aus der Ordnung der Dermaptera z.B. Forficula auricularis.
Aus der ordnung der Isoptera z.B. Reticulitermes spp..
Aus der Ordnung der Anoplura z.B. Pediculus humanus corporis.
Aus der Ordnung der Heteroptera z.B. Cimex lectularius, Rhodnius prolixus, Triatoma infestans.
Aus der Ordnung der Lepidoptera z.B. Ephestia kuehniella, Galleria mellonella.
Aus der Ordnung der Coleoptera z.B. Anobium punctatum, Rhizopherta dominica, Hylotrupes bajulus, Oryzaephilus surinamensis, Sitophilus spp., Dermestes spp., Trogoderma spp., Anthrenus spp., Lyctus spp., Niptus hololeucus, Gibbium psylloides, Tribolium spp..
Aus der Ordnung der Hymenoptera z.B. Monomorium pharaonis, Losius niger, Vespa spp..
Aus der Ordnung der Diptera z.B. Aedes aegypti, Anopheles spp., Culex spp., Musca spp., Fannia spp., Calliphora sppl, Lucilia spp., Chrysomyia spp., Stomoxys spp., Tabanus spp..
Aus der Ordnung der Siphonaptera z.B. Xenopsylla cheopis, Ceratophyllus spp..

Der erfindungsgemäße Wirkstoff kann allein oder in Gemisch mit anderen Insektiziden, wie Phosphorsäureestern, Carbamaten, Pyrethroiden oder Arylpyrazolen angewendet werden.

Beim Gemisch mit anderen Insektiziden seien folgende beimischbare Komponenten beispielhaft aufgeführt.

Phosphorsäureester: Dichlorvos (DDVP), Fenitrothion, Malathion, Chlorpyrifos, Diazinon, Methylpyrimiphos.

Carbamate: Propoxur, Carbofuran, Carbaryl und Bendiocarb.

Pyrethroide: Cyfluthrin, Tetramethrin, Allethrin, Vaporthrin, Tetrallethrin, Bioresmethrin, Esbiol, Cypermethrin, Alphamethrin, Decis, Permethrin.

Bei der Kombination des erfindungsgemäßen Wirkstoffes mit einem oder mehreren insektiziden Wirkstoffen der Reihe Phosphorsäureester, Carbamaten und weiteren Pyrethroiden kann gegebenenfalls eine synergistische Wirkungssteigerung erzielt werden.

Wie aus dem Beispiel A und Tabelle 1 ersichtlich, konnte beispielsweise bei der Kombination des erfindungsgemäßen Wirkstoffes mit Dichlorvos (DDVP) eine synergistische Wirkungssteigerung erzielt werden.

Weiterhin konnte bei der Kombibation des erfindungsgemäßen Wirkstoffes mit Propoxur und Cyfluthrin ein synergistischer Effekt erreicht werden.

Für die Herstellung gebrauchsfertiger Formulierungen wird der Wirkstoff allein oder in Kombination mit anderen in die üblichen Formulierungen überführt wie Lösungen, Emulsionen Makro- und Mikroemulsionen, Spritzpulver, Suspensionen, Puder, Stäubemittel, Schäume, Pasten Aerosole, Ölsprühmittel, Suspensionskonzentrate, Wirkstoff-imprägnierte Natur-und synthetische Stoffe ins besondere sogenannte slow release-Formulierungen, die den Wirkstof langsam in dosierter Menge abgeben, Feinstverkapselungen von polymeen Stoffen, Brennsätze, Räucherpatronen, Räucherdosen, Mückenspiralen, ULV-, Kalt- und Warmnebelformulierungen, Mottenpapiere sowie Verdampferplättchen für die Anwendung auf elektrisch oder chemothermisch erhitzten Vorrichtungen.

Die Formulierungen werden in bekannter Weise hergestellt, z.B. durch Vermischen des Wirkstoffes mit Streckmitteln, also flüchtigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylen oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon und Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normler Temperatur und unter Normaldruck gasförmig sind, z.B. Aerosoltreibgase sowie Halogenkohlenwasserstoffe wie Butan, Propan, Stickstoff und Kohlendioxid als feste Trägerstoffe seien aufgeführt: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte Steine wie Calcit, Marmor, Bims, Sepiolith, Dolomit sowie synthetische Granulate aus anorganischem und organischen Mehlen sowie Granulate aus organischen Material wie Sägemehl, Kokosnußschalen, Maiskolben, und Tabakstengel; als Emulgier- und/oder schaumerzeugende Mittel; nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen auch Haftmittel wie Carboxymethylcellulose, natürliche und synthetische pulvrige, körnige und latexförmige Polymere verwendet werden, wie Gummi arabicum, Polyvinylakohol, Polyvinylacetat.

Handelsübliche Fertigformulierungen oder die zur Weiterverdünnung vorgesehenen Konzentrate enthalten im allgemeinen 0,005 bis 96 Gew.-% Wirkstoff, vorzugsweise zwischen 0,02 und 90 %.

Der Wirkstoffgehalt der aus den handelsübilchen Formulierungen bereiteten Anwendungsformen kann in weiten Bereichen variieren. Die Wirkstoffkonzentration der Anwendungsformen kann von 0,001 bis 100 Gew.-% Wirkstoff, vorzugsweise zwischen 0,01 und 20 Gew.-% liegen.

Die Anwendung geschieht in einer den Anwendungsformen angepaßten üblichen Weise.

Besonders bevorzugt sind Sprayformulierungen und Verdampferplättchen.

Beispielhaft seien die folgenden Formulierungsbeispiele aufgeführt.

Hierbei werden die nachstehend gekennzeichneten Wirkstoffe I bis VI eingesetzt.

Wirkstoffe

I

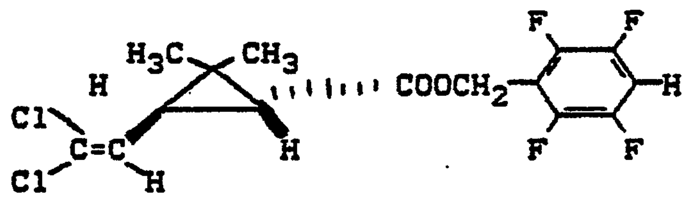

(erfindungsgemäß) (+)1R-trans

II

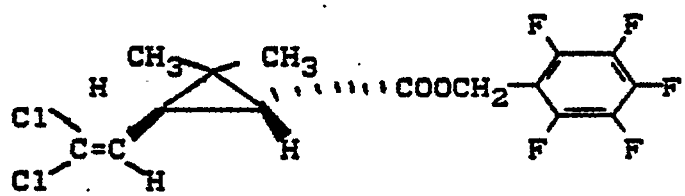

((-)1R-trans-Isomeres des Fenfluthrins)

III

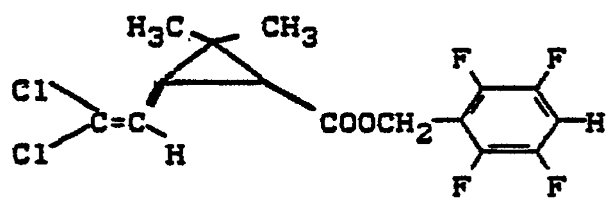

(±)cis/trans-Gemisch

IV

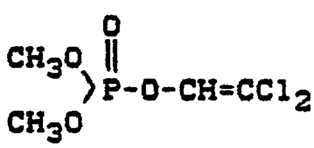

(Dichlorvos)

V

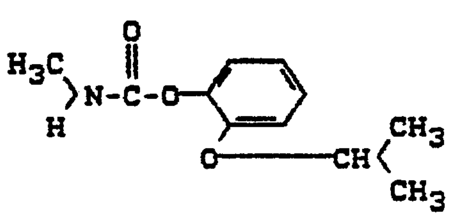

(Propoxur)

VI 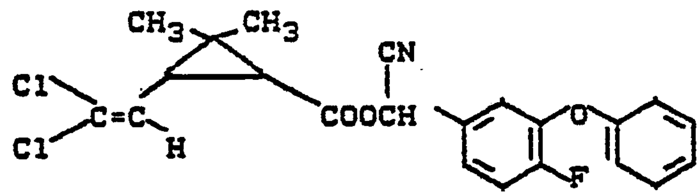

(Cyfluthrin) (±)cis/trans-Gemisch

Formulierungsbeispiele

| 1. Sprayformulierung | Gewichtsteile in % |
|---|---|
| Wirkstoff I | 0,04 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 94,85 |
| 2. Sprayformulierung | |
| Wirkstoff II | 0,04 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 94,85 |
| 3. Sprayformulierung | |
| Wirkstoff III | 0,04 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 94,85 |

| 4. Sprayformulierung | Gewichtsteile in % |
| --- | --- |
| Wirkstoff IV | 1,0 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 93,89 |
| 5. Sprayformulierung | |
| Wirkstoff I | 0,04 |
| Wirkstoff IV | 1,0 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 93,85 |
| 6. Sprayformulierung | |
| Wirkstoff II | 0,04 |
| Wirkstoff IV | 1,0 |
| Desodoriertes Kerosen/Gemisch gesättigter, aliphatischer Kohlenwasserstoffe | 5,0 |
| Parfümöl | 0,01 |
| Stabilisator | 0,1 |
| Treibmittel: Propan/Butan 15:85 | 93,85 |

| 7. Sprayformulierung | Gewichtsteile in % |
|---|---|
| Wirkstoff V | 1,0 |
| Wirkstoff VI | 0,025 |
| Desodoriertes Kerosen/Gemisch gesättigter, aliphatischer Kohlenwasserstoffe | 38,36 |
| Parfümöl | 0,03 |
| Stabilisator | 0,1 |
| Methylenchlorid | 15,0 |
| Treibmittel: Propan/Butan 15:85 | 45,485 |

| 8. Sprayformulierung | Gewichtsteile in % |
|---|---|
| Wirkstoff V | 1,0 |
| Wirkstoff VI | 0,025 |
| Wirkstoff I | 0,04 |
| Desodoriertes Kerosen/Gemisch gesättigter, aliphatischer Kohlenwasserstoffe | 38,36 |
| Parfümöl | 0,03 |
| Stabilisator | 0,1 |
| Methylenchlorid | 15,0 |
| Treibmittel: Propan/Butan 15:85 | 45,445 |

| 9. Sprayformulierung | Gewichtsteile in % |
|---|---|
| Wirkstoff V | 1,0 |
| Wirkstoff VI | 0,025 |
| Wirkstoff II | 0,04 |
| Desodoriertes Kerosen/Gemisch gesättigter, aliphatischer Kohlenwasserstoffe | 38,36 |
| Parfümöl | 0,03 |
| Stabilisator | 0,1 |
| Methylenchlorid | 15,0 |
| Treibmittel: Propan/Butan 15:85 | 45,445 |

| 10. Verdampferplättchen | |
|---|---|
| Wirkstoff I | 10, 20 oder 30 mg |
| Diisononylphthalat | 150 mg |
| Parfüm | 0,25 mg |
| Celluloseplättchen (16 × 28 × 3 mm) | 800 mg |

| 11. Verdampferplättchen | |
|---|---|
| Wirkstoff II | 10 mg |
| Diisononylphthalat | 150 mg |
| Parfüm | 0,25 mg |
| Celluloseplättchen (16 × 28 × 3 mm) | 800 mg |

Beispiel A

In Räumen von 30 $m^3$ Rauminhalt werden pro Versuch 3 Drahtkäfige mit je 20 resistenten Musca domestica-Männchen aufgehängt. Dann werden die Sprühdosen in den Raum gesprüht, die gemäß Formulierungsbeispielen 1-9 die Wirkstoffe I, II und III oder die Wirkstoffgemische I+III; II+III; IV+V; IV+V+I und IV+V+II enthalten.

Die ausgebrachte Sprühmenge pro Spraydose beträgt 12,4 g. Nach dem Sprühen werden die Räume geschlossen und die Wirkung der Sprühnebel auf die Fliegen durch Fenster laufend beobachtet. Protokolliert wird, nach wievielen Minuten 50 und nach wievielen Minuten 95 % der Tiere in die Rückenlage gefallen sind (Knock down-Effekt). Nach 1 Stunde Testdauer wird abschließend der Prozentsatz der

Knock down-gegangenen Tiere ermittelt. Die ermittelten Werte enthält die nachfolgende Tabelle.

Tabelle 1

| Aerosol-Test (Musca domestica, resistent) | | | | |
|---|---|---|---|---|
| Wirkstoffe | Ausgebrachte Wirkstoffmenge mg/30m$^3$ | 50% knock down nach Minuten | 95% knock down nach Minuten | % knock down nach 1 Stunde |
| I | 5 | 22′ | 47′ | 96 |
| I | 7,5 | 18′ | 33é | 99 |
| II | 5 | 19′ | 37é | 99 |
| III | 5 | 38′ | 52é | 97 |
| IV | 124 | – | – | 22 |
| I + IV | 5 + 124 | 18′ | 39é | 97 |
| I + IV | 7,5 + 124 | 14′ | 24é | 99 |
| II + IV | 5 + 124 | 15′ | 26é | 99 |
| V + VI | 124 + 3,1 | 18′ | 27é | 100 |
| V + VI + I | 124 + 3,1 + 5 | 12′ | 17é | 100 |
| V + VI + II | 124 + 3,1 + 5 | 10′ | 15é | 100 |

Beispiel B

Auf die Heizplatte kleiner Elektroverdampferöfchen, die Temperaturen von 130 und 160°C erzeugen, werden kleine wirkstoffhaltige Celluloseplättchen gemäß Formulierungsbeispielen Nr. 9 und 10 gelegt. Die Geräte werden in gleichgroßen und gleich ausgestatteten Wohnräumen über eine Steckdose mit dem Stromnetz verbunden und aufgeheizt.

Während der Versuche blieb ein Fenster der Räume nach außen in Kippstellung geöffnet. Sofort nach Inbetriebnahm der Öfchen wurden in jedem Raum 2 Drahtkörbchen mit je 20 Mücken der Art Aëdes aegypti, von 3-4 Tagen Alter, gehängt. Eine halbe bzw. eine Stunde später wurde die knock down-Wirkung auf die Mücken kontrolliert. Nach längerer Brenndauer der Öfchen wurden zu bestimmten Zeiten erneut frische Mücken in der gleichen Weise in die Räume gebracht und wieder nach einer halben oder einer Stunde auf Wirksmkeit getestet. Temperatur der Heizöfchen, Wirkstoffmengen, Brenndauer, Testzeit und knock down-Wirkung gehen aus der nachfolgenden Tabelle hevor.

(% Know down: Prozentsatz der Mücken, die in die Rückenlage gefallen sind).

Indem vorliegenden Test wurden die Wirkstoffe I (erfindungsgemäß) und II ((-)1R-trans Isomeres des Fenfluthrins) eingesetzt.

Tabelle 2

| Dampftest (Aedes aegypti) | | | | | |
|---|---|---|---|---|---|
| Wirkstoff I Menge in mg | Heizöfchen-temperatur °C | Tiere eingesetzt nach Brenndauer der Öfchen in Stunden | Verweildauer der Tiere im Raum in Stunden | % knock down Wirkstoff I | % knock down Wirkstoff II (10 mg) |
| 10 | 160 | 0 | 1 | 100 | 100 |
| | | 8 | 1 | 100 | 100 |
| | | 26 | 1 | 55 | 40 |
| 10 | 130 | 0 | 1 | 100 | 100 |
| | | 8 | 1 | 100 | 100 |
| | | 26 | 1 | 100 | 100 |
| | | 50 | 1 | 85 | 97 |
| 20 | 160 | 0 | 1 | 100 | 100 |
| | | 8 | 1 | 100 | 100 |
| | | 28 | 1 | 100 | 92 |
| 20 | 130 | 0 | 1 | 100 | 100 |
| | | 8 | 1 | 100 | 100 |
| | | 26 | 1 | 100 | 100 |
| | | 50 | 1 | 100 | 95 |

Tabelle 2 Fortsetzung

| Wirkstoff I Menge in mg | Heizöfchen-temperatur °C | Tiere eingesetzt nach Brenndauer der Öfchen in Stunden | Verweildauer der Tiere im Raum in Stunden | % knock down Wirkstoff I | % knock down Wirkstoff II (10 mg) |
|---|---|---|---|---|---|
| 30 | 160 | 0 | 0,5 | 100 | 45 |
| | | 8 | 0,5 | 100 | 100 |
| | | 28 | 0,5 | 100 | 92 |
| | | 32 | 0,5 | 100 | 47 |
| 30 | 130 | 0 | 0,5 | 97 | 65 |
| | | 8 | 0,5 | 100 | 100 |
| | | 26 | 0,5 | 100 | 97 |
| | | 50 | 0,5 | 92 | 75 |

Herstellungsbeispiele

(+) 1R-trans-2,2-Dimethyl-3-(2,2-dichlorvinyl)-cyclopropancarbonsäure-2,3,5,6-tetrafluor-benzylester (andere Bezeichnung: (+) (1R,3S-)-3-(2,2-Dichlorvinyl)-2,2-dimethylcyclopropancarbonsäure-2,3,5,6-tetrafluorbenzylester)

Variante a):

227 g (1 Mol) (+)1R-trans-Permethrinsäurechlorid (optische Reinheit: 95 %) wird bei 40°C zu 180 g (1 Mol) 2,3,5,6-Tetrafluorbenzylalkohol getropft. Gegen Ende der Gasentwicklung wird zur Vervollständigung der Umsetzung auf 100°C erhitzt und anschließend wird das Reaktionsprodukt destilliert. $Kp_{0,15}$: 135°.

Man erhält 352,4 g (95 % der Theorie) der Titelverbindung vom Schmelzpunkt 32°C, [α]= +15,3° (C=0,5 $CHCl_3$) IR-Daten: 3080, 2965, 2935, 2895, 1735, 1620, 1510, 1465, 1430, 1395, 1385, 1345, 1290, 1270, 1230, 1180, 1120, 1025, 1050, 995 850-950, 785.

Variante b):

27 g (0,11 Mol) Kalium-(+)1R-trans-2,2-dimethyl-3-dichlorvinyl-cyclopropancarboxylat und 20 g (0,1 Mol) 2,3,5,6-Tetrafluorbenzylchlorid sowie 0,005 Mol Pentamethylethylentriamin werden in 70 ml Acetonitril 5 Stunden gekocht, bis die halogenverbindung völlig verbraucht ist.

Anschließend engt man am Rotationsverdampfer ein, nimmt mit Petrolether auf, schüttelt mit Wasser aus und erhält nach Einengen der organischen Phase 367,3 g (90 % der Theorie) der Titelverbindung.

Schmelzpunkt sowie physikalische Daten siehe oben.

**Patentansprüche**

1. (+)1R-trans-2,2-Dimethyl-3-(2,2-dichlorvinyl)-cyclopropancarbonsäure-2,3,5,6-tetrafluorbenzylester der Formel

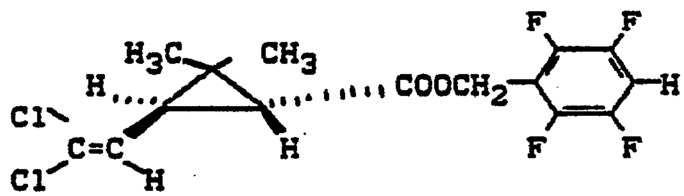

(I)

2. Verfahren zur Herstellung von (+)1R-trans-2,2-Dimethyl-3-(2,2-dichlorvinyl)-cyclopropancarbonsäure-2,3,5,6-tetrafluorbenzylester der Formel

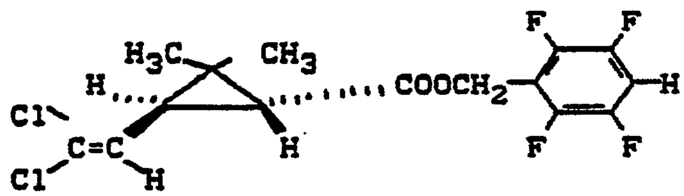

(I)

dadurch gekennzeichnet, daß man
a) entweder
(+)1R-trans-Permethrinsäurechlorid der Formel

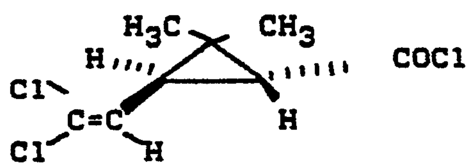

(II)

mit 2,3,5,6-Tetrafluorbenzylalkohol der Formel

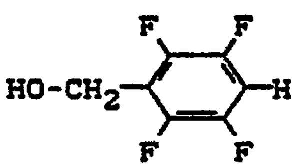

(III)

bei Temperaturen zwischen 20 und 100°C umsetzt,
oder
b) das Salz der (+)1R-trans-Permethrinsäure der Formel

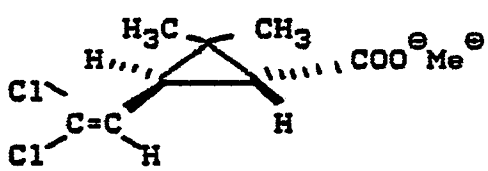

(IV)

worin Me⊕ für ein einwertiges Kation steht,
mit einer Verbindung der Formel

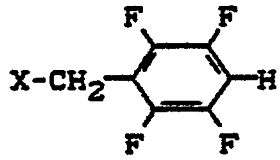

(V)

worin
X für einen anionisch abspaltbaren Rest steht,
umsetzt.

3. Verfahren gemäß Anspruch 2, dadurch gekennzeichnet, daß bei der Verfahrensvariante b) in Formel (III) Me für ein Alkalimetallkation und in der Formel (IV) X für Halogen steht.

4. Insektizide Mittel, gekennzeichnet durch einen Gehalt an (+)1R-trans-2,2-Dimethyl-3-(2,2-dichlorvinyl)-cyclopropancarbonsäure-2,3,5,6-tetrafluorbenzylester der Formel

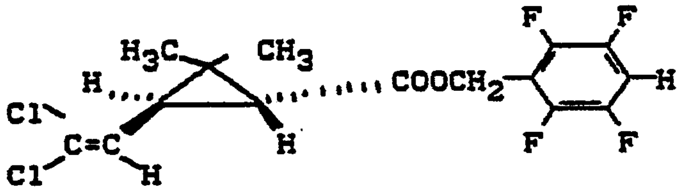

(I)

5. Insektizide Mittel nach Anspruch 4, dadurch gekennzeichnet, daß sie neben (+)1R-trans-2,2-Dimethyl-3-(2,2-dichlorvinyl)-cyclopropancarbonsäure-2,3,5,6-tetrafluorbenzylester einen oder mehrere weitere insektizide Wirkstoffe der Reihe Phosphorsäureester, Carbamat und weitere Pyrethroide enthalten.

6. Insektizide Mittel nach Anspruch 4-5, dadurch gekenzeichnet, daß sie neben (+)1R-trans-2,2-Dimethyl-3-(2,2-dichlorvinyl)-cyclopropancarbonsäure-2,3,5,6-tetrafluorbenzylester einen oder mehrere weiter insektizide Wirkstoffe der Reihe Dichlorvos (DDVP), Propoxur und Cyfluthrin enthalten.

7. Verfahren zur Bekämpfung von Insekten, dadurch gekenzeichnet, daß man (+)1R-trans-2,2,-Dimethyl-3-(2,2-dichlorvinyl)-cyclopropancarbonsäure-2,3,5,6-tetrafluorbenzylester der Formel

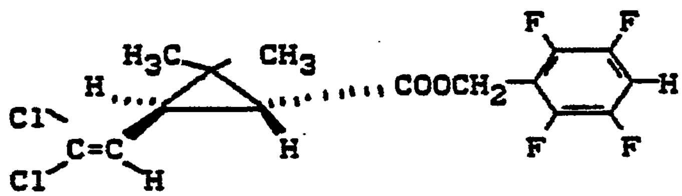

(I)

auf Insekten und/oder ihren Lebensraum einwirken läßt.

8. Verwendung von (+)1R-trans-2,2-Dimethyl-3-(2,2-dichlorvinyl)-cyclopropancarbonsäure-2,3,5,6-tetrafluorbenyzlester der Formel

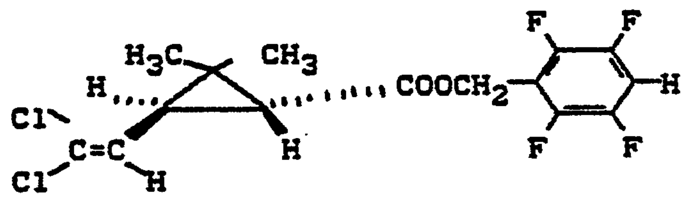

(I)

zur Bekämpfung von Insekten.

9. Verfahren zur Herstellung von insektiziden Mitteln, dadurch gekennzeichnet, daß man (+)1R-trans-2,2-Dimethyl-3-(2,2-dichlorvinyl)-cyclopropancarbonsäure-2,3,5,6-tetrafluorbenzylester der Formel

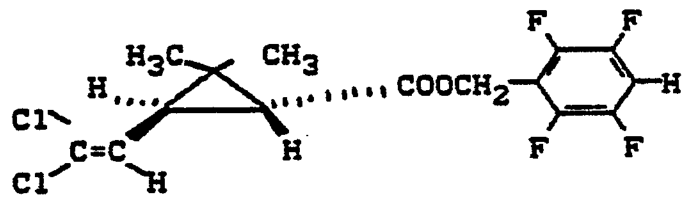

(I)

mit Streckmitteln und/oder oberflächenaktiven Mitteln vermischt.

## Claims

1. 2,3,5,6-Tetrafluorobenzyl (+)1R-trans-2,2-dimethyl-3-(2,2-dichlorovinyl)-cyclopropanecarboxylate of the formula

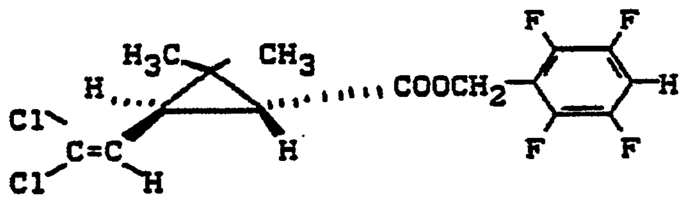 (I)

2. Process for the preparation of 2,3,5,6-tetrafluorobenzyl (+)1R-trans-2,2-dimethyl-3-(2,2-dichlorovinyl)-cyclopropanecarboxylate of the formula

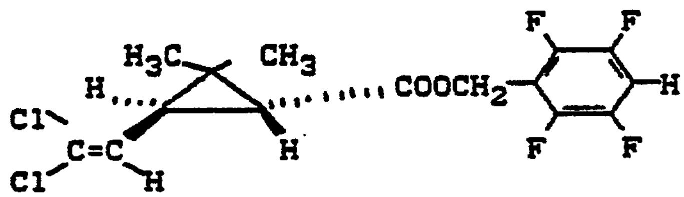 (I)

characterized in that
a) either
(+)1R-trans-permethrin acid chloride of the formula

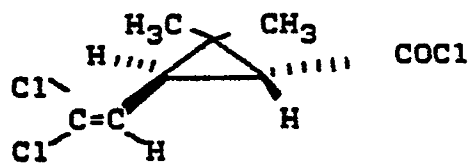 (II)

is reacted with 2,3,5,6-tetrafluorobenzyl alcohol of the formula

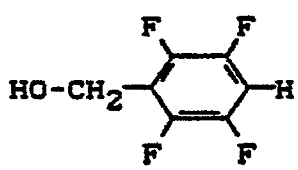 (III)

at temperatures between 20 and 100°C, or
b) the salt of (+)1R-trans-permethrin acid of the formula

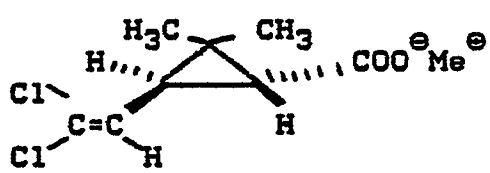 (IV)

in which Me⊕ represents a monovalent cation, is reacted with a compound of the formula

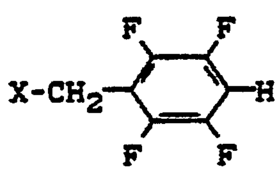 (V)

in which X represents an anionically removable radical.

3. Process according to Claim 2, characterized in that, in process bariant b) Me in formula (III) represents an alkali metal cation, and X in the formula (IV) represents halogen.

4. Insecticides, characterized in that they contain 2,3,5,6-tetrafluorobenzyl (+)1R-trans-2,2-dimethyl-3-(2,2-dichlorovinyl)-cyclopropanecarboxylate of the formula

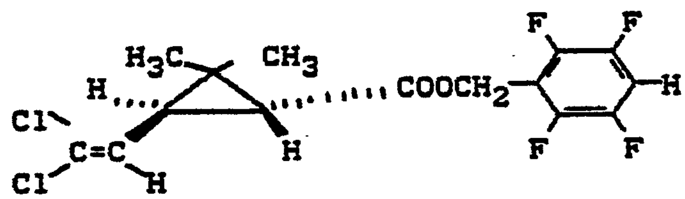 (I)

5. Insecticides according to Claim 4, characterized in that they contain, besides 2,3,5,6-tetrafluorobenzyl (+)1R-trans-2,2-dimethyl-3-(2,2-dichlorovinyl)-cyclopropane-carboxylate, one or more further insecticidal active compounds from the series comprising phosphoric acid esters, carbamates and further pyrethroids.

6. Insecticides according to Claim 4–5, characterized in that they contain, besides 2,3,5,6-tetrafluorobenzyl (+)1R-trans-2,2-dimethyl-3-(2,2-dichlorovinyl)-cyclopropane-carboxylate, one or more further insecticidal active compounds from the series comprising dichlorvos (DDVP), propoxur and cyfluthrin.

7. Process for combating insects, characterized in that 2,3,5,6-tetrafluorobenzyl (+)1R-trans-2,2-dimethyl-3-(2,2-dichlorovinyl)-cyclopropanecarboxylate of the formula

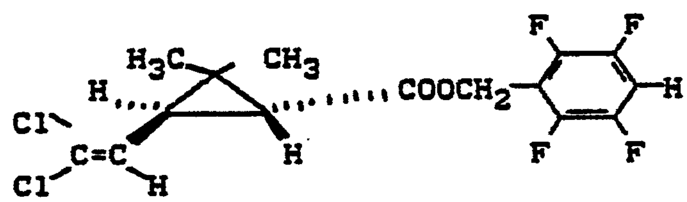 (I)

is allowed to act on insects and/or on their environment.

8. Use of 2,3,5,6-tetrafluorobenzyl (+)1R-trans-2,2-dimethyl-3-(2,2-dichlorovinyl)-cyclopropanecarboxylate of the formula

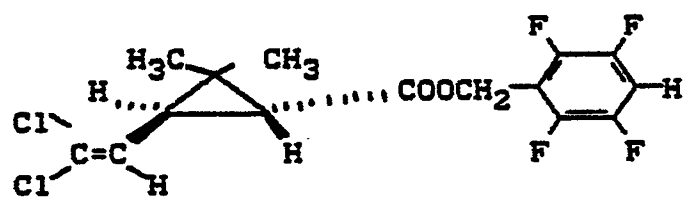 (I)

for combating insects.

9. Process for the preparation of insecticides, characterized in that 2,3,5,6-tetrafluorobenzyl (+)1R-trans-2,2-dimethyl-3-(2,2-dichlorovinyl)-cyclopropanecarboxylate of the formula

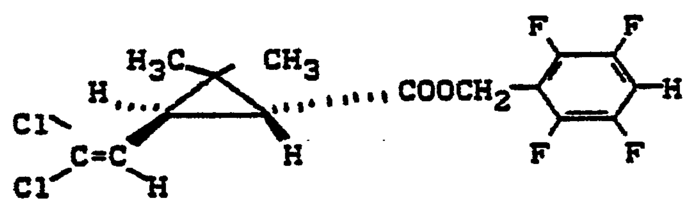 (I)

is mixed with extenders and/or surface-active agents.

**Revendications**

1. Ester 2,3,5,6-tétrafluorobenzylique de l'acide (+)1R-trans-2,2-diméthyl-3-(2,2-dichlorovinyl)-cyclopropanecarboxylique de formule

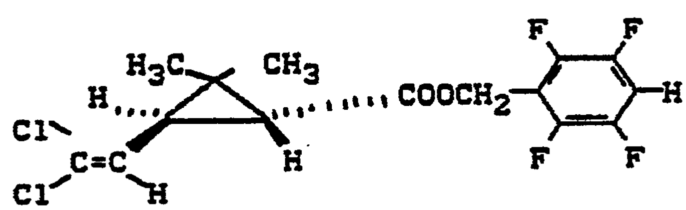 (I)

2. Procédé de préparation de l'ester 2,3,5,6-tétrafluorobenzylique de l'acide (+)1R-trans-2,2-diméthyl-3-(2,2-dichlorovinyl)-cyclopropanecarboxylique de formule

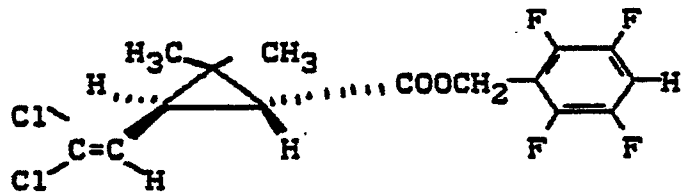

(I)

caractérisé en ce que

a) on fait réagir le chlorure d'acide de (+)1R-trans-perméthrine de formule

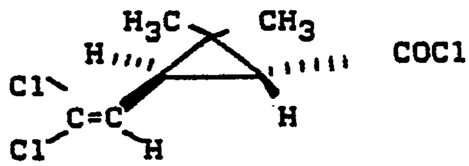

(II)

avec l'alcool 2,3,5,6-tétrafluorobenzylique de formule

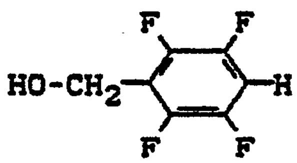

(III)

à des températures comprises entre 20 et 100°C, ou bien

b) on fait réagir le sel de l'acide de (+)1R-trans-perméthrine de formule

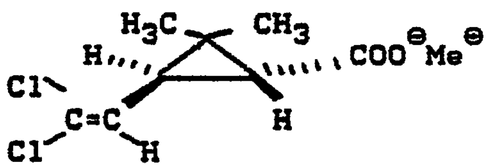

(IV)

dans laquelle Me⊕ désigne un cation monovalent, avec un composé de formule

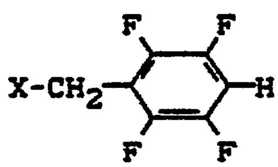

(V)

dans laquelle X est un reste éliminable par voie anionique.

3. Procédé suivant la revendication 2, caractérisé en ce que, dans la variante b) du procédé, le symbole Me dans la formule (III) représente un cation de métal alcalin et le symbole X dans la formule (IV) représente un halogène.

4. Compositions insecticides, caractérisées par une teneur en ester 2,3,5,6-tétrafluorobenzylique d'acide (+)1R-trans-2,2-diméthyl-3-(2,2-dichlorovinyl)-cyclopropanecarboxylique de formule

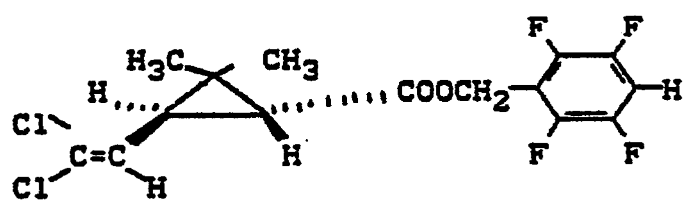

(I)

5. Compositions insecticides suivant la revendication 4, caractérisées en ce qu'elles contiennent à côté d'ester 2,3,5,6-tétrafluorobenzylique d'acide (+)1R-trans-2,2-diméthyl-3-(2,2-dichlorovinyl)-cyclopropanecarboxylique, une ou plusieurs autres substances douées d'activité insecticide de la série des esters d'acide phosphorique, des carbamates et d'autres pyréthroïdes.

6. Compositions insecticides suivant les revendications 4 et 5, caractérisées en ce qu'elles contiennent, à côté de l'ester 2,3,5,6-tétrafluorobenzylique d'acide (+)1R-trans-2,2-diméthyl-3-(2,2-dichlorovinyl)-cyclopropanecarboxylique, une ou plusieurs autres substances douées d'activité insecticide, de la série du Dichlorvos (DDVP), du Propoxur et de la Cyfluthrine.

7. Procédé pour combattre des insectes, caractérisé en ce qu'on fait agir l'ester 2,3,5,6-tétrafluorobenzylique de l'acice (+)1R-trans-2,2-diméthyl-3-(2,2-dichlorovinyl)-cyclopropanecarboxylique de formule

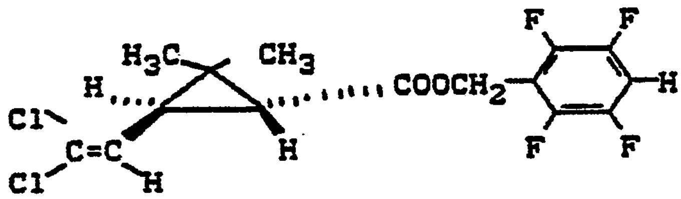 (I)

sur les insectes et/ou sur leur milieu.

8. Utilisation de l'ester 2,3,5,6-tétrafluorobenzylique de l'acide (+)1R-trans-2,2-diméthyl-3-(2,2-dichlorovinyl)-cyclopropanecarboxylique de formule

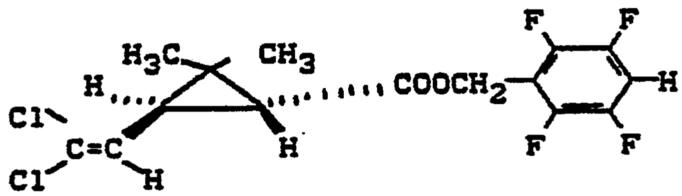 (I)

pour combattre des insectes.

9. Procédé de préparation de compositions insecticides, caractérisé en ce qu'on mélange de l'ester 2,3,5,6-tétrafluorobenzylique d'acide (+)1R-trans-2,2-diméthyl-3-(2,2-dichlorovinyl)-cyclopropanecarboxylique de formule

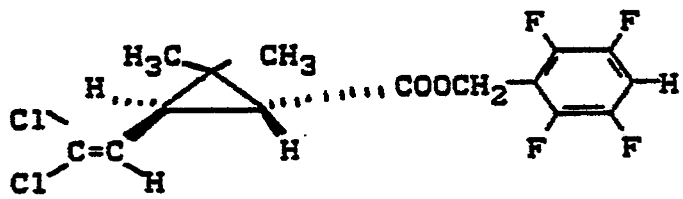 (I)

avec des diluants et/ou des agents tensio-actifs.